# EUROPEAN PATENT SPECIFICATION

(11) **EP 2 358 653 B1**
(45) Date of publication and mention of the grant of the patent: **22.07.2015**
(21) Application number: 09744163.8
(22) Date of filing: 03.11.2009
(51) Int. Cl.: C07C 1/207, C07C 1/24, C07C 9/06, C07C 9/08, C07C 11/04, C07C 11/06, C07C 11/08, C07C 45/52, C07C 47/22

(54) **A PROCESS FOR PREPARING LOWER HYDROCARBONS FROM GLYCEROL**
VERFAHREN ZUR HERSTELLUNG NIEDERER KOHLENWASSERSTOFFE AUS GLYCERIN
PROCÉDÉ POUR LA PRÉPARATION D'HYDROCARBURES INFÉRIEURS À PARTIR DE GLYCÉROL

(30) Priority: 05.11.2008 US 111388 P; 14.11.2008 SE 0850077
(43) Date of publication of application: 24.08.2011
(73) Proprietor: BioFuel-Solution AB, 200 61 Limhamn (SE)
(72) Inventor: HULTEBERG, Christian, S-217 46 Malmö (SE); BRANDIN, Jan, S-215 79 Malmö (SE)
(74) Representative: Ström & Gulliksson AB
(86) International application number: PCT/EP2009/064517
(87) International publication number: WO 2010/052208

(56) References cited:
- WO-A2-2006/087083
- US-A1- 2003 220 531
- SHEKHAR R ET AL: "Decarbonylation and hydrogenation reactions of allyl alcohol and acrolein on Pd(110)" SURFACE SCIENCE, NORTH-HOLLAND PUBLISHING CO, AMSTERDAM, NL LNKD- DOI:10.1016/0039-6028(94)90597-5, vol. 319, no. 3, 10 November 1994 (1994-11-10), pages 298-314, XP025774067 ISSN: 0039-6028 [retrieved on 1994-11-10] cited in the application

## Description

### Field of the invention

The present invention relates to a method for producing hydrocarbons, such as alkanes or alkenes, from oxygenated hydrocarbons by the use of catalysts.

### Background

There is a growing interest and need for renewable fuels throughout the world to replace fossil fuels. Furthermore, there is also a need for unsaturated lower hydrocarbons not originating from petroleum for use as starting materials in the chemical industry. Lower unsaturated hydrocarbons, not originating from petroleum, could also find us as monomers within the polymer industry.

Glycerol or glycerin, C₃H₈O₃, is a viscous uncolored transparent liquid in pure state at ambient conditions. The glycerol has traditionally been a byproduct from soap manufacturing, using animal and vegetable oils and fats. Today the majority of the produced glycerol originates from the production of biodiesel, such as methyl esters of fatty acids, by transesterfication of the same types of triglycerides with an alcohol, for instance methanol. The amount of glycerol produced is approximately 1:9 by weight of the biodiesel produced.

Due to the growing interest in renewable fuels for transportation, the production of biodiesel has increased tremendously over the last 10 years and as a result, the glycerol market has been saturated and the value of glycerol has decreased radically. Because of the abundance and low cost, the interest in glycerol as a fuel has grown. The glycerol has a heating value (heat of combustion) of 16 MJ/kg, but is due to the high flame point (180°C) difficult to burn. Thereby use of glycerol as a fuel requires support fuel and specially designed burners.

Accordingly, there is need for a process to convert glycerol into a form suitable for use as fuel.

US 2003/0220531 A1 discloses a method of producing hydrocarbons from oxygenated hydrocarbon reactants, such as sorbitol. The disclosed method allows the production of a mixture of hydrocarbons by the liquid-phase reaction of water with sorbitol.

The method disclosed in US 2003/0220531 A1 results in a complex mixture of different alkanes including the potent green house gas methane. Thus, there is a need of a further separation step to individualize the separate alkanes. Furthermore, the conversion ratio of the feedstock is moderate.

### Summary

Accordingly, embodiments of the present invention seeks to mitigate, alleviate, circumvent or eliminate one or more of the above-identified deficiencies and to provide a method of producing a hydrocarbon comprising two or three carbon atoms from glycerol, wherein the hydrocarbon only comprises carbon and hydrogen atoms. For this purpose the method comprises the steps of:
providing glycerol;
converting glycerol to acrolein through use of a first catalyst, catalyzing dehydration reactions; and
converting the formed acrolein to the hydrocarbon comprising two or three carbon atoms, through use of a second catalyst catalyzing de-carbonylation reactions, or through use of a second catalyst catalyzing hydrogenation reactions and a third catalyst catalyzing de-hydration reactions.

Especially, in such a method said hydrocarbon may be ethane, said first catalyst may be an acidic catalyst, such as a catalyst comprising WO₃ on ZrO₂, said second catalyst may be a catalyst, catalyzing water-gas-shift (WGS), hydrogenation and de-carbonylation reactions, such as a catalyst comprising Pt on CeO₂ and glycerol may be provided as a mixture with water, such as a mixture having a glycerol concentration of 15 to 30wt%.

Further advantageous features of the invention are defined in the dependent claims and with regard to embodiments disclosed herein.

### Brief description of the drawings

Fig 1. Depicts an experimental set-up employed.
Fig 2 and 3. Depicts the yield of ethane, CO₂/CO and propanol produced from glycerol with external addition of hydrogen.
Fig 4. Depicts the yield of ethane, CO₂, CO and propanoic acid produced from glycerol without external addition of hydrogen.
Fig. 5 Depicts the yield of ethene produced from glycerol
Fig. 6 Depicts the yield of propene and propane produced from glycerol
Fig. 7 Depicts the yield of propane produced from glycerol

### Description

### Definitions:

Within the following description the following definitions apply:
Yield is adopted from IUPAC Compendium of Chemical Terminology (http://goldbook.iupac.org/index.html) book and is intended to mean the fraction of the amount of chemical compound following a specified chemical reaction.
As used herein, the term "hydrocarbon" relates to compounds only comprising carbon and hydrogen atoms. Without limitation, such compounds comprise alkanes, alkenes and alkynes.

### Embodiments:

An embodiment of the present invention relates to a method of producing hydrocarbons comprising two or three carbon atoms from glycerol, wherein the hydrocarbon only comprises carbon and hydrogen atoms. Such a method comprises the steps of: providing glycerol; converting glycerol to acrolein through use of a first catalyst, catalyzing de-hydration reaction; and converting the formed acrolein to the hydrocarbon comprising two or three carbon atoms, trough the use of a second catalyst catalyzing de-carbonylation reactions, or through use of a second catalyst catalyzing hydrogenation reactions and a third catalyst catalyzing de-hydration reactions.

As stated above, glycerol is converted to acrolein through the use of a first catalyst. Typically the conversion takes place in gas phase. The first catalyst is selected from catalysts known to the skilled artesian to catalyze dehydration reactions, such as the dehydration of glycerol to form acrolein.

According to one embodiment, the conversion of glycerol to acrolein and the conversion of acrolein to the hydrocarbon are performed in gas phase.

In WO 2006/087084 and US 5,387,720, several aspects of the conversion of glycerol in gas phase to acrolein are disclosed. Furthermore, several examples of suitable catalysts are disclosed.

According to WO 2006/087084 examples of catalysts catalyzing dehydration reactions, such as the dehydration of glycerol to form acrolein, may be chosen from natural or synthetic siliceous materials or acidic zeolites; mineral supports, such as oxides, coated with mono-, di-, tri- or polyacidic inorganic acids; oxides or mixed oxides, or alternatively heteropolyacids. The catalyst may be chosen from zeolites, Nafior^{®} composites (based on sulfonic acid of fluorinated polymers), chlorinated aluminas, phospho- tungstic and/or silicotungstic acids and acid salts, and various solids of metal oxide type such as tantalum oxide Ta₂O₅, niobium oxide Nb₂O₅, alumina Al₂O₃, titanium oxide TiO₂, zirconia ZrO₂, tin oxide SnO₂, silica SiO₂ or silico-aluminate SiO₂-Al₂Oa, impregnated with acidic functions such as borate BO₃, sulfate SO₄, tungstate WO₃, phosphate PO₄, silicate SiO₂ or molybdate MoO₃.

According to another embodiment the first catalyst comprises an acidic active phase, such as a mineral acid, a solid acid, an acidic zeolite or an acidic metal oxide:
According to another embodiment the active phase of the first catalyst comprises a mineral acid, such as H₃PO₄, H₂SO₄, H₃BO₃. The active phase may also comprise a solid acid, such as alumina (Al₂O₃), titanium dioxide (TiO₂). Furthermore, the solid acid may be an acidic zeolite, i.e. the H-form of zeolites, such as H-mordenite, H-Beta, H-ZSM-5 etc) or an acidic transition metal oxide, such as molybdic acid (MoO₃*H₂O), tungstic acid (WO₃*H₂O), and vanadic acid (H₃VO₄).

The active phase may be mounted or deposited on a carrier material. If the active phase comprises a mineral acid, then mounting of the active phase on the carrier material is to be preferred. If the active phase comprises a metal oxide then deposition of the active phase on the carrier material is to be preferred. The carrier material may comprise or even consist of silica (SiO₄), Alumina (Al₂O₃), carbon or ZrO₂.

According to one embodiment, the first catalyst comprises or even consist of WO;^{*}H₂0 supported on ZrO₂. WO₃ on ZrO₂ was shown be efficient in producing acrolein from glycerol, with an almost complete conversion of glycerol to acrolein with no or very few side-products. This catalyst also shows good long term stability with small deposition of carbon and is commercially available for other usage.

When the first catalyst comprises or even consist of WO₃*H₂O supported on ZrO₂ the conversion of glycerol to acrolein may be maximized, as the yield of acrolein determines the possible yield of the hydrocarbon.

In converting glycerol to acrolein, glycerol may be provided as a mixture with water. Preferably, the glycerol/water-mixture may be provided in gas phase. Accordingly, the glycerol/water-mixture may be preheated and/or evaporated before glycerol is converted to acrolein.

According to one embodiment, glycerol is converted to acrolein in a first reactor, comprising the first catalyst. In such an embodiment glycerol may be provided to the first reactor, comprising the first catalyst, as a mixture with water. Preferably, the glycerol/water-mixture is provided to the reactor, comprising the first catalyst, in gas phase. Accordingly, the glycerol/water-mixture may be preheated and/or evaporated before being provided to the first reactor.

Furthermore, the dehydration of glycerol to form acrolein is an endothermic reaction and it may thus be advantageous to provide glycerol, optionally mixed with water, preheated.

The content of glycerol in the glycerol/water-mixture may vary. The content of glycerol may be 1 to 75wt%, such as 10 to 50wt% or 15 to 30wt%.

The concentration of glycerol should not be too high, so as to avoid spurious reactions, such as the formation of glycerol ethers, or residues such as tar like components and coke; or reactions between the acrolein produced and the glycerol. Moreover, the glycerol solution should not be too dilute on account of the energy needed to preheat and evaporate of glycerol/water-mixture.

The concentration of the glycerol solution may be adjusted by recycling the water produced by the reaction. In order to reduce the glycerol transportation and storage costs, glycerol may be provided as a concentrated aqueous solution of 40 to 100 wt% glycerol, dilution to the optimum content before conversion to acrolein being performed by recycling some of the steam and/or water produced in various reaction(s) of the method.

The temperature of the glycerol/water-mixture, when provided may be 100 to 600°C, such as 200 to 400°C, or 250 to 350°C.

The glycerol may be converted to acrolein at a temperature of 100 to 600°C, such as 200 to 400°C or 250°C to 350°C. Preferably, glycerol is converted to acrolein at a temperature of 250°C to 350°C. Within this temperature range the selectivity towards acrolein is improved.

In an embodiment comprising a first reactor comprising the first catalyst, the first reactor may be provided with temperature control means, such as heating and cooling means. According to one embodiment the first reactor is equipped with internal heating and/or cooling surface(s). Such surface(s) may be used to improve the reaction conditions by maintaining the temperature within the reactor comprising the first catalyst at the desired temperature, such as in the range 250°C to 350°C.

The temperature of the glycerol/water-mixture, when provided, may be lower than the temperature at which glycerol is converted to acrolein. Furthermore, the temperature of the glycerol/water-mixture, when provided, may also be higher than the temperature at which glycerol is converted to acrolein.

In one embodiment, the temperature of the glycerol/water-mixture, when provided to a reactor comprising the first catalyst, may be lower than the temperature within the reactor comprising the first catalyst. Furthermore, the temperature of the glycerol/water-mixture, when provided to a reactor comprising the first catalyst, may also be higher than the temperature within the reactor comprising the first catalyst.

According to one embodiment, the overall heat balance and/or the overall heat balance of a first reactor, comprising the first catalyst, will determine if it is preferred that the temperature of the glycerol/water-mixture, when provided, is lower than the temperature at which glycerol is converted to acrolein or if it is preferred that the temperature of the glycerol/water-mixture, when provided, is higher than the temperature at which glycerol is converted to acrolein.

As the conversion dehydration of glycerol is an endothermic process, heat has to be supplied in order to maintain a constant temperature for the conversion of glycerol to acrolein. One way of providing heat is to supply heat from other exothermic processes, such as hydrogenation reactions. Accordingly, heat from exothermic process(es) may be transferred through use of heat exchange.

In an embodiment comprising a first reactor comprising the first catalyst, heat has to be supplied to the reactor, if the temperature within said reactor is to be maintained, as the dehydration of glycerol is an endothermic process. One way of providing heat is to supply heat from other exothermic processes, such as hydrogenation reactions. Accordingly, heat from exothermic process(es) may be transferred through use of heat exchange.

If more than one process takes place in the same reactor, the need for transferring heat from an exothermic to the endothermic hydration process may be eliminated or at least reduced. Similarly such an arrangement may reduce or even eliminate the cooling needs for an exothermic process.

According to one embodiment, the conversion of glycerol to acrolein is performed in gas phase. The glycerol is then preferably converted to acrolein at a pressure of 0.1 to 100 bar, such as 1 to 40 bar, or 2 to 10 bar.

According to one embodiment, the conversion of glycerol to acrolein is performed in gas phase at a temperature of 250°C to 350°C and a pressure of 2 to 10 bar.

Various process technologies may be used in operating a reactor comprising one or more catalysts. As known to the skilled artesian, such technologies, without limitations, include a fixed-bed process, a fluidized-bed process or a circulating fluidized-bed process.

The selection of the optimum process is on various criteria; the fixed-bed process has the advantage of simplicity; the fluidized-bed process makes it possible to continuously discharge the spent catalyst and to permanently recharge fresh catalyst without stopping the production, with the possibility of being isothermic. The circulating fluidized-bed process has the advantage of optimizing the reaction selectivity by permanently returning freshly regenerated catalyst into the reactor, while at the same time compensating for the energy exchange between the reactor and the regenerator.

The first catalyst may be generated through treatment be oxygen, hydrogen or by other treatments such as washing with solvents and/or treatment with H₂O₂.

Glycerol to be used in the process may originate from production of bio-diesel, such as production of methyl esters of fatty acids from acylglycerols. As there is an increasing need for non-fossil fuels, the production of methyl esters of fatty acids, and thus also the by-product glycerol, has increased.

One alternative to the use of glycerol originating from the trans-esterfication or hydrolysis of tri-glycerides, in order to obtain fatty acids or esters of these, is to use glycerol produced by algae or microorganisms. By utilizing glycerol-producing algae or glycerol-producing bacteria or yeast, glycerol may be produced from carbon dioxide and sunlight (algae or protozoa) or from sugar or cellulosic materials (microorganisms).

As glycerol may be fed to the reactor, comprising the first catalyst, as a mixture with water, there is no need to separate glycerol from the broth, in which the glycerol is produced by glycerol-producing algae or glycerol-producing microorganism, such as yeast. Instead the hydrocarbon comprising two or three carbon atoms may be separated from water. Such separation will typically be much more convenient, as there will be a larger difference in boiling point.

As one example, glycerol may be produced from CO₂ and sunlight by use of algae from e.g. the Dunaliella family (Aharon Oren. "A hundred years of Dunaliella research:1905-2005". Saline System 2005, 1:2 (doi:10.1186/1746-1448-1-2))

This may be done in either type of reactor, such as an open pond or photo-bioreactor. The photo-bioreactor is preferred due to higher productivity.

Furthermore, carbon dioxide entrapped by algae may, at least partly, originate from carbon dioxide produced as a by-product in various processes according to embodiments of the present invention. Such processes may include water gas shift reactions and combustions to generate heat.

As glycerol producing algae cultures require high saline water concentrations to survive, there will be no contaminations e.g. from competing algae as they will not survive. Furthermore, there will not be any competition for sweet water or costs for water desalinification. Glycerol from the algae residue may be separated using osmotic pressure (rapid decrease of solvent salinity).

Furthermore, there are several microorganisms that produce glycerol from various feedstocks.

For instance *Pichina Farinosa* is known to produce glycerol from glucose. A complete list of glycerol producing yeast, mold, algae, protozoa and bacteria may be found in: Taherzadeh, M.J., Adler, L., Lidén, G:. Strategies for enhancing fermentative production of glycerol - a review. Enzyme and Microbial Technology . 2002, Vol. 31. The reported tolerated concentrations in the broth are about 15 to 30 wt%, i.e. they are very well suited to be fed directly to the reactor comprising the first catalysts, once the microorganisms have been removed, such as filtered of. The microorganisms may be removed by thermal decomposition of the broth and a simple filtration to remove the residues.

The elimination of the need to separate the produced glycerol from water is important, as glycerol recovery has been a limiting factor in the commercialization of fermentation processes. Furthermore, the high yields and concentrations imply that the production of glycerol will be more advantageous compared to ethanol fermentation in the production of hydrocarbons suitable as fuels from renewable resources. In an analogue fashion to the ethanol, the glycerol may use cellulosic biomass as a substrate, following the similar pretreatment methods as in ethanol fermentation, but with different microorganisms.

One embodiment of the present invention relates to use of a mixture of glycerol and water produced by algae and/or microorganisms as feedstock in producing least one hydrocarbon comprising two or three carbon atoms, wherein the hydrocarbon only comprises carbon and hydrogen atoms.

According to one embodiment, the acrolein formed through use of the first catalyst may be converted to at least one hydrocarbon comprising two or three carbon atoms, wherein the hydrocarbon only comprises carbon and hydrogen atoms, by use of a second catalyst, or by use of a second, a third and optionally a fourth catalyst.

The second catalysts may be selected from catalysts catalyzing hydrogenation, de-carbonylation and/or water-gas-shift reactions.

The third catalyst may be selected from catalysts catalyzing de-hydration reactions and/or hydrogenation reactions.

The fourth catalyst may be selected from catalysts catalyzing hydrogenation reactions.

Hydrogenations are reactions, wherein compounds are reduced by addition of elementary hydrogen. Typically, hydrogenation reaction includes addition of hydrogen over a carbon-carbon double or triple bond. Furthermore it may also be the addition of hydrogen over a carbon-oxygen double bond, i.e. a carbonyl, such reduction of an aldehyde or a keton to form a primary or secondary alcohol, respectively.

De-carbonylation reactions are reactions wherein a carbon atom with at least one bond to at least one oxygen, e.g. carbonyl carbon, such as a formyl or carboxy group, is cleaved of. The carbon may be cleaved of as carbon monoxide or as carbon dioxide.

According to one embodiment, de-carbonylation reactions relates to the removal of a formyl group from a molecule, usually by emitting carbon monoxide or carbon dioxide.

According to another embodiment de-carbonylation reactions relates to the removal of a carboxyl group, usually by emitting carbon dioxide. Such removal of a carboxyl group may also be denoted de-carboxylation. De-carboxylation reactions may also relate to reactions, wherein the carbonyl and the bond alcohol part of the carboxylic acid ester is cleaved of.

According to another embodiment, de-carbonylation reactions also relates to the cleavage of a primary alcohol, to form a compound comprising one carbon less, such as forming ethane from propanol.

Water gas shift reaction (WGS) is a chemical reaction in which carbon monoxide reacts with water to form carbon dioxide and hydrogen.

De-hydration reactions are reactions wherein water is eliminated from a molecule. One common example of a de-hydration reaction is the conversion of an alcohol to the corresponding alkene. Another example is the conversion of glycerol to acrolein. This conversion actually includes the elimination of two moles of water for each mol of acrolein formed.

According to one embodiment the second catalyst may be selected from catalysts catalyzing de-carbonylation reactions. A catalyst catalyzing de-carbonylation reactions may be used to form ethene from acrolein. Ethene may be hydrogenated to ethane, if the second catalyst in addition to de-carbonylation reactions also catalyzes hydrogenation reactions and if hydrogen is provided. If the second catalyst doesn't catalyzes hydrogenation reactions, a third catalyst catalyzing hydrogenation reactions may be used to hydrogenated ethene to form ethane, if hydrogen is provided. Hydrogen may be provided by a WGS-reaction, shifting the carbon monoxide cleaved and water to hydrogen and carbon dioxide.

According to one embodiment the second catalyst may be selected from catalysts catalyzing water-gas-shift (WGS), hydrogenation and de-carbonylation reactions. One example of such a catalyst was surprisingly shown to be a catalyst comprising Pt on CeO₂. The ratio of Pt to CeO₂ may be 0.1 to 10 wt%, such as 1 to 5 wt%. Furthermore, at least 75 wt%, such as at least 85 wt% or even at least 95 wt%, of the second catalyst may be Pt on CeO₂.

Pt on CeO₂ is known to catalyze WGS-reactions. However, the catalysis of the de-carbonylation reaction was a surprising finding. Furthermore, Pt is well-known to catalyze hydrogenation reactions. As Pt on CeO₂ was shown to both catalyze de-carbonylation and WGS-reactions, there is no need to supply external hydrogen for the hydrogenation of ethene to form ethane. The carbon monoxide formed in the de-carbonylation of acrolein, will be shifted to hydrogen in the presence of water.

One embodiment of the present invention relates to a method of converting glycerol to ethane or ethene. In such an embodiment the second catalyst should catalyze de-carbonylation reactions.

One embodiment of the present invention relates to a method of converting glycerol to ethane. In such a method glycerol is first converted to acrolein through use of a first catalyst catalyzing de-hydration reactions, such as a catalyst comprising WO₃ supported on ZrO₂. The thus formed acrolein is then converted to ethane through use of a second catalyst catalyzing water-gas-shift (WGS), hydrogenation and de-carbonylation reactions, such as a catalyst comprising Pt on CeO₂.

Without being bond to any theory it is believed that acrolein first is de-carbonylated to ethene and carbon monoxide (CO). CO and water are then reacted to form hydrogen and carbon dioxide, i.e. a water-gas-shift reaction. The formed hydrogen is then consumed in the hydrogenation of ethene to formed ethane.

By using a second catalyst catalyzing water-gas-shift (WGS), hydrogenation and de-carbonylation reactions, ethane may be produced from glycerol in at least 60%, such as at least 80%, yield. As the carbon monoxide formed in the de-carbonylation of acrolein is shifted to hydrogen and carbon dioxide, no toxic carbon monoxide is formed, and there is no need for any external hydrogen supply. Furthermore, ethane is, in contrast to glycerol and other lower alcohols, easily separated from water.

The major byproduct in forming ethane from glycerol, as disclosed above, will be propanoic acid. It may be formed in less than 10% yield from glycerol. Without being bond to any theory it is believed, that propanol is formed from a reaction between hydrogen, formed through a WGS-reaction, and acrolein.

Shekar et al, in Surface Science 319, 1994, 298-314, have disclosed experimental findings relating to decarbonylation and hydrogenations of pure allyl alcohol and of pure acrolein on Pd(110) in very small experimental scale by use of a single crystal of Pd(110). They conclude that, in contrast to previous results for allyl alcohol, there is no evidence for C-O scission of any C3 oxygente on Pd(110).

If using a second catalyst catalyzing water-gas-shift (WGS), hydrogenation and de-carbonylation reactions and providing hydrogen, in addition to glycerol, the major byproduct will be propanol and carbon monoxide. Without being bond to any theory it is believed, that propanol is formed from a reaction between hydrogen and acrolein. Propanol may be converted to propane or propene.

As the de-hydration of glycerol to acrolein is an endothermic reaction, while the hydrogenation of ethene to ethane is an exothermic reaction, it may be advantageous to have both the first and the second catalyst present in a reactor, being the same reactor.

According to another embodiment, glycerol may be converted to ethane in first reactor comprising a first and a second catalyst. The first catalyst may be an acidic catalyst, such as catalyst comprising WO₃ on ZrO₂, catalyzing de-hydration reactions. The second catalyst may be a catalyst, catalyzing water-gas-shift (WGS), hydrogenation and de-carbonylation reactions, such as a catalyst comprising Pt on CeO₂. The temperature in the first reactor may be 250°C to 350°C. Furthermore, the pressure may be about 3.5 to 4.5 bar, such as about 4 bar. Glycerol may be provided to the first reactor as a mixture with water having a glycerol concentration of 15 to 30wt%. Furthermore, the temperature of the glycerol/water-mixture when provided to the first reactor may be 250 to 350 °C. The temperature of the glycerol/water-mixture when provided to the first reactor may be lower than the temperature in the first reactor. Further, the first and said second catalyst are present in the same reactor. In such an embpdiment ethane may be produced in at least 80% yield from glycerol.

If a catalyst catalyzing de-carbonylation reactions, but not, or at least to a very limited extent, water-gas-shift (WGS) and/or hydrogenation reactions, is utilized as the second catalyst, ethene may be produced instead of ethane. According to one embodiment, limited extent is intended to mean that less than 10%, such as less than 5% or even less than 1% of the formed ethene is hydrogenated to form ethane. Examples of catalysts catalyzing de-carbonylation reactions, but not, or at least to a very limited extent, water-gas-shift (WGS) and/or hydrogenation reactions are catalysts comprising iridium or palladium, such as catalysts comprising Pd/CeO₂, as the catalyzing specie. To control the process, the major control parameter is the catalyst composition and the operating temperature. The operating temperature may be 100 to 600°C, such as 200 to 400°C or 250°C to 350°C. Preferably, acrolein is converted to ethene at a temperature of 250°C to 350°C. The yield of ethene may be produced from glycerol in at least 40%, such as least 50% or even at least 60% yield.

In such a process one of the major byproducts will be carbon monoxide, which may be used to produce hydrogen in a separate WGS-reaction. Carbon monoxide and hydrogen may also be used to produce hydrocarbons, e.g. in a Fischer-Tropsch process.

In an embodiment, wherein the second catalyst catalyzes de-carbonylation, the temperature, at which acrolein is converted to ethane and/or ethene, may be 100 to 600°C, such as 200 to 400°C or 250°C to 350°C. Furthermore, the pressure at which, acrolein is converted to ethane and/or ethene, may be 0.1 to 100 bar, such as 1 to 40 bar, or 2 to 10 bar.

One embodiment of the present invention relates to a method of converting glycerol to propane or propene. In such a method the second catalyst may be selected from catalysts catalyzing hydrogenation reactions. Furthermore, such a method may also comprise a third catalyst, which may be selected from catalysts catalyzing de-hydration reactions. Optionally, such a method also comprises a fourth catalyst, which may be selected from catalysts catalyzing hydrogenation reactions.

According to one embodiment, which relates to a method of converting glycerol to propane or propene, the second catalyst may be selected from catalysts catalyzing hydrogenation reactions. Preferably, such a second catalyst should not catalyze de-carbonylation reactions or at least catalyze de-carbonylation reactions to a very limited extent. According to one embodiment, limited extent is intended to mean that less than 5%, such as less than 1% or even less than 0.1% of the acrolein is de-carbonylated. One example among others of such a catalyst, which catalyzes hydrogenation reactions, but to very limited extent de-carbonylation reactions, is catalysts comprising Cu on ZnO₂. These catalysts should be of such a nature that they activate the reaction of hydrogen with the desired bond to be saturated, without activating the cleavage of the carbon-carbon bond in question. In such an embodiment acrolein may be converted to propanol, if hydrogen is provided to the second catalyst. The thus formed propanol may then be converted to propane or propene through use of additional catalysts, such as third, fourth or even fifth catalyst.

According to one embodiment catalysts catalyzing hydrogenation reactions may be selected from catalysts comprising a platinum group metal (Pt, Pd, Rh, Ru, Os and Ir), Ni or Cu. The catalyst may be supported or unsupported. Suitable supports include alumina, silica, carbon, spinel, and other such materials. Suitable hydrogenation catalysts are manufactured by BASF Catalysts (Iselin, NJ), Johnson Matthey (London, England), Sud-Chemie (Munich, Germany), Topsoe (Lyngby, Denmark) and others.

By use of a dehydration catalyst, such as a catalyst comprising WO₃ on ZrO₂, as the third catalyst, propanol may be dehydrated to propene.

According to one embodiment, the third catalyst, catalyzing dehydration reactions, is an acidic catalyst. According to another embodiment the active phase of the third catalyst comprises a mineral acid, such as H₃PO₄, H₂SO₄, H₃BO₃. The active phase may also comprise a solid acid, such as alumina (Al₂O₃), titanium dioxide (TiO₂). Furthermore, the solid acid may be an acidic zeolite, i.e. the H-form of zeolites, such as H-mordenite, H-Beta, H-ZSM-5 etc) or an acidic transition metal oxide, such as molybdic acid (MoO₃*H₂O), tungstic acid (WO₃*H₂O), and vanadic acid (H₃VO₄). As disclosed above, propanol may also be formed as a byproduct in embodiments, wherein ethane is the main product, and may thus also be dehydrated to propene.

While propanol, having nearly the same boiling point as water, is hard to separate from water, propene is easily separated from water. From a fuel perspective, the gaseous fuels (ethane/propane) are preferable, as they do not have corroding properties compared to the corresponding alcohols.

In a process wherein propene is to be formed, the formation of propane as by-product may be reduced by providing hydrogen in a molar ration between glycerol and hydrogen of 1:4, such as 1:3 or 1:2.5, i.e. slight stoichiometric excess. Hydrogen may also be provided in stoichiometric amount.

Subsequently, propene may be hydrogenated to form propane, by use of a fourth catalyst catalyzing hydrogenating reaction and by providing hydrogen.

In a process wherein propane is to be formed, the yield of propene as by-product may be reduced by providing hydrogen in stoichiometric excess with regard to glycerol. The molar ration between glycerol and hydrogen, when hydrogen is to be provided in stoichiometric excess, should be more than 1:3, such as 1:10, 1:8 or 1:6.

While propanol, having nearly the same boiling point as water, is hard to separate from water, propane and propene are easily separated from water

In an embodiment, wherein the second catalyst catalyzes hydrogenation reactions, the temperature, at which acrolein is hydrogenated, may be 100 to 600°C, such as 200 to 400°C or 250°C to 350°C. Furthermore, the pressure at which, acrolein is hydrogenated, may be 0.1 to 100 bar, such as 1 to 40 bar, or 2 to 10 bar.

In an embodiment, wherein the third catalyst catalyzes de-hydration reactions, the temperature, at which propanol is de-hydrated, may be 100 to 600°C, such as 150 to 400°C or 150°C to 350°C. Furthermore, the pressure at which, propanol is de-hydrated, may be 0.1 to 100 bar, such as 1 to 40 bar, or 2 to 10 bar.

In an embodiment, wherein the fourth catalyst catalyzes hydrogenation reactions, the temperature, at which propene is hydrogenated, may be 100 to 600°C, such as 150 to 400 °C or 150 °C to 350 °C. Furthermore, the pressure at which propene is hydrogenated, may be 0.1 to 100 bar, such as 1 to 40 bar, or 2 to 10 bar.

Hydrogen used in different embodiments disclosed herein, may be provided by steam reformation of glycerol.

One embodiment, relates to a method as disclosed herein, which further comprises the step of providing hydrogen.

According to one embodiment catalysts catalyzing water gas shift reactions may be selected from catalysts based on Fe/Cr, Cu/ZnO, Pt/Ce, and sulfides of Co/Mo. Different catalysts catalyzing water gas shift reactions result in different temperature requirements for the reaction. For example, typical operating temperatures when Fe/Cr catalyst is employed are in excess of about 350°C, while those for Cu/ZnO are typically from about 200°C to about 300°C, and those for Pt/Ce are typically from about 200°C to about 500°C. Some of the catalysts require the presence of additional chemicals or agents to maintain activity. For example, sulfides of Co/Mo require the presence of sulfur in the reaction stream to maintain activity. In certain embodiments, these agents can be added to the reaction mixture, they can be carried over from an earlier processing step with one of the reagents, such as for example, sulfates or sulfuric acid being present in a glycerol feedstream after acid catalyzed transesterification with sulfuric acid, or the agent can be left out, and provision made for periodic regeneration of the catalyst. The agent can be removed in a downstream process step, or left in the product stream. In some embodiments, the agent is preferably recycled. As the catalysts are identified, their temperature requirements can be readily ascertained, and employed within the process for producing alcohol.

If the method according to embodiments disclosed herein, comprises more than one reactor, hydrogen may be provided to any or all of these reactors. If hydrogenation, takes place in a reactor subsequent to the first reactor, it may be advantageous to provide hydrogen to this reactor and not to the first reactor, in order to avoid reactions forming by-products in the first reactor.

Furthermore, it may be advantageous to have the possibility to control the pressure individually over different reactors if more than one.

The first, the second and possibly further catalysts may be combined in different ways.

In one embodiment the first and second catalyst are present in the same reactor, such as two layers in a catalytic bed. The second catalyst may also be gradually mixed in the first catalyst.

In a process wherein the first catalyst catalyzes an endothermic reaction while the second one catalyzes an exothermic reaction, heat transfer between the reactions will be very efficient the two catalysts are present in the same reactor.

While it is possible to have the first and the second catalyst on the same carrier, it is preferred to have different carriers. As the catalytic properties is affected both by the catalytic metal as well as the carrier, its preferred to use different carriers. By having different carriers it is possible to achieve more selective catalysis and thus reduce the formation of by-products.

Although, the different catalysts are present on separate support, it may be, as disclosed above, advantageous to utilize the catalysts in mechanically mixed forms, as it promotes heat transfer.

Also further catalysts may be combined in different ways with respect to each other but also with respect to the first and the second catalyst.

In one embodiment the first and second catalyst are present in the same reactor, such as two layers in a catalytic bed. Furthermore, the third and the fourth catalyst are present in the same reactor, but separate from the first and second catalyst.

In one embodiment the first catalyst is present in a first reactor and the second and third catalyst in a second reactor, such as two layers in a catalytic bed. The third catalyst may also be gradually mixed in the second catalyst.

In one embodiment the first catalyst is present in a first reactor, the second catalyst is present in a second reactor and the third and the fourth catalyst in a third reactor, such as two layers in a catalytic bed. The fourth catalyst may also be gradually mixed in the third catalyst.

Ethane, ethene, propane and propene are all useful as fuels. Accordingly, one embodiment relates to a method of producing ethane, ethene, propane and/or propene from glycerol.

Especially ethene and propene are useful as monomers in preparing polymers. Accordingly, one embodiment relates to method of producing ethene and/or propene from glycerol.

The heating value of ethane and propane is somewhat higher than the corresponding value of the ethene and propene. Accordingly, one embodiment relates to method of producing ethane and/or propane from glycerol.

Another embodiment relates to the use of a hydrocarbon, such as ethane, ethene, propane and/or propene, obtained by a process disclosed herein, as a fuel. Such a fuel may be burned to generate heat. Furthermore, it may be used as fuel in vehicles.

Another embodiment relates to the use of an unsaturated hydrocarbon, such as ethene and/or propene, obtained by a process disclosed herein, as a monomer in preparing polymers.

The conversion ratio of glycerol may be more than 80%, such as more than 90%, more than 95%, or even more than 99%. The conversion ratio of glycerol may essentially quantitative, i.e. glycerol is converted to below the limit of detection.

Glycerol may be converted to a mixture of different hydrocarbons comprising two or three carbon atoms. However, it is preferred if the main product is produced in more than 50% yield, such as more than 65% yield or even more than 80% yield from glycerol.

The production of a single hydrocarbon comprising two or three carbon atoms, such as ethane, ethene, propane or propene, in a high yield will facilitate the purification of the product mixture, comprising at least one hydrocarbon comprising two or three carbon atoms and water.

Furthermore, while the separation of glycerol from water is energy consuming and difficult, due to the hydrophilic properties and the high boiling point of glycerol, the separation of hydrocarbons according to embodiments disclosed herein, such as ethane, ethane, propane are propene, from water is convenient. Typically water will be condensed and thereby separated from the volatile hydrocarbon(s). The heat released, may be used to heat for example the glycerol/water-mixture prior to being fed to the reactor comprising the first catalyst.

In contrast to lower monohydric alcohols, hydrocarbons comprising two or three carbon atoms, wherein the hydrocarbon only comprises carbon and hydrogen atoms, are easily separated from water. Accordingly, it is highly advantageous to have a process wherein glycerol is converted to a hydrocarbon if the product is to be used as a fuel.

The produced hydrocarbon may be separated from by-products through methods known to the skilled artesian. Such methods include, distillation, stripping, pressure swing adsorption, ammine scrubbing and combinations thereof.

According to one embodiment, hydrogen present in the product stream comprising at least one hydrocarbon comprising two or three carbon atoms, wherein the hydrocarbon only comprises carbon and hydrogen atoms, may be recycled. Such recycling may be achieved through us of pressure swing adsorption technology.

Also, by-products may be recycled.

As disclosed above, water present in the product stream may be recycled. Furthermore, it may be used to dilute the glycerol or glycerol/water mixture prior to glycerol is being converted to acrolein.

Furthermore, hydrogen present in the product stream comprising at least one hydrocarbon comprising two or three carbon atoms may be selectively burnt off using a catalyst, such as Pt/ Al₂O₃, and oxygen. The heat generated may be used to heat various streams within process of various embodiments.

In the claims, the term "comprises/comprising" does not exclude the presence of other species or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality.

### Examples

### Abbreviations

| | |
|---|---|
| FID | Flame Ionization Detector |
| GC | Gas Chromatograph |
| TCD | Thermal Conductivity Detection |
| WGS | water-gas-shift |

The examples given below are only intended to further illustrate the invention and are by no means intended to limit the scope of the invention as defined by the appended claims.

### General

The feed in all the examples below consisted of an aqueous solution of 20 wt% glycerol or 10 wt% acrolein. The solution was stored in a pressurized vessel and the inlet flow rate was controlled by a mass flow controller. The inlet liquid flow was heated and vaporized in a pre-heater. At this point, nitrogen or hydrogen gas could be introduced into the stream to facilitate the vaporization.

Depending on the actual experiment, one or more of the three reactors in series were used (see further below). The catalyst in the different reactors may be the same or different. Similarly, the space velocity within the different reactors may be the same or different

After leaving the final reactor, the gas was cooled down and condensed in a water-cooled condenser. Thereafter a liquid sample was collected for analysis. The uncondensable gas was measured by a digital gas flow meter and a gas sample was collected in sample bags.

The liquid analysis was performed using a Varian CP-3800 gas chromatograph equipped with an 1177 split/split-less injector, a CP-wax 58/FFAP column and a flame ionization detector. The permanent gas analysis was performed using a Varian CP-4900 gas chromatograph, using both a mol-sieve 5A PLOT column and a PoraPlot column with two TCD detectors.

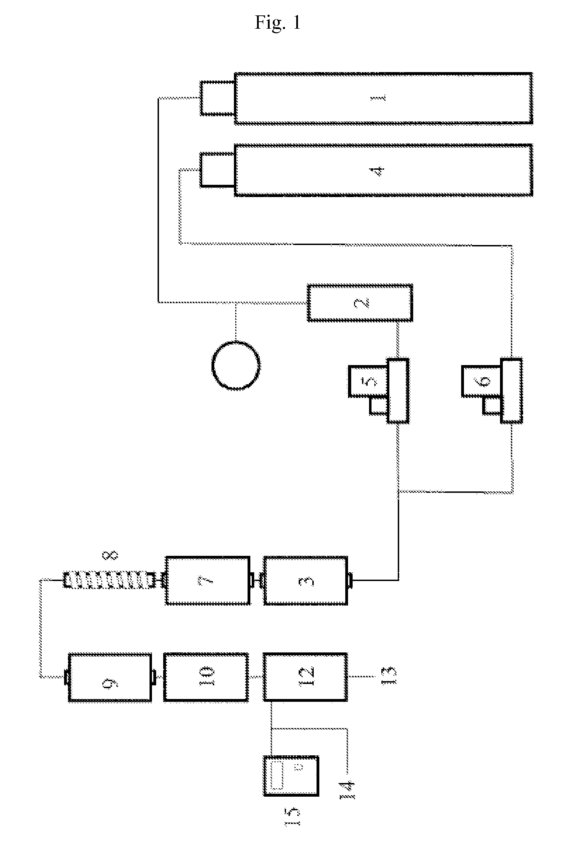

An outline of the experiment set-up is depicted in Fig. 1. The set-up comprises a nitrogen reservoir 1 connected to a pressurized vessel 2 comprising the glycerol/water mixture or the acrolein/water mixture to be fed to a pre-heater 3. A hydrogen reservoir 4 is also connected to the pre-heater 3. Both the connection of the hydrogen reservoir 4 and the pressurized vessel 2 are controlled by mass flow controllers 5 and 6. The pre-heater 3 is connected to a first reactor 7. The first reactor is connected to a second reactor 8, which is connected to a third reactor 9. The third reactor is connected to a condenser 10, which may cool down the outlet flow of the reactors. The outlet flow may be divided into a liquid stream 13 and gas stream 14 in a chiller 12. The flow of the gas stream may be measured may be measured by a gas flow meter 15.

The pre-heater 3 had a length of 250 mm and diameter of 12 mm

The first reactor 7, being a tube-reactor, had a length of 250 mm and diameter of 25 mm.

The second reactor 8, being a tube-reactor, had a length of 250 mm and diameter of 12 mm and was equipped with heat-tape.

The third reactor 9, being a tube-reactor, had a length of 250 mm and diameter of 35 mm.

The preheater 3 and all the reactors 7 to 9 were equipped with thermocouples at their in- and out-lets.

### Exarnaple 1 - production of ethane with external hydrogen supply

Glycerol was converted to ethane over a catalyst bed with two layers of different catalysts as disclosed below.

In the first layer 56 g of a first catalyst, consisting of 10 wt% of WO₃ supported on ZrO₂ in grains of the size 20-30 mesh, was used. On top of this, another layer with 15 g of a second catalyst consisting of 4 w% Pt on CeO₂ was applied as grains of the size 20-30 mesh.

The inlet liquid stream consisted of 20 wt% of glycerol in water fed to the pre-heater 3. A gas stream of 400 ml/min of hydrogen was also fed to the pre-heater 3. The liquid stream was preheated and evaporated, to 300 °C, prior entering the first reactor 7. The inlet of reactor 1 was held at 320 °C and a pressure of 4 bar gauge was applied over the reactors 7-9. The outlet stream was cooled down in the condenser 10 and the water was condensed in a chiller. The liquid stream 13 was collected in a sample vessel, while the gas stream 14 was collected in a Tedlar gas bag. The liquid sample was analyzed with a GC equipped with FID and a WAX-column for hydrocarbons (propanol, propanal, propanoic acid etc.). The gas sample was analyzed with a two channel GC equipped with TCD for analyzing CO, CO₂, ethene, ethane etc.

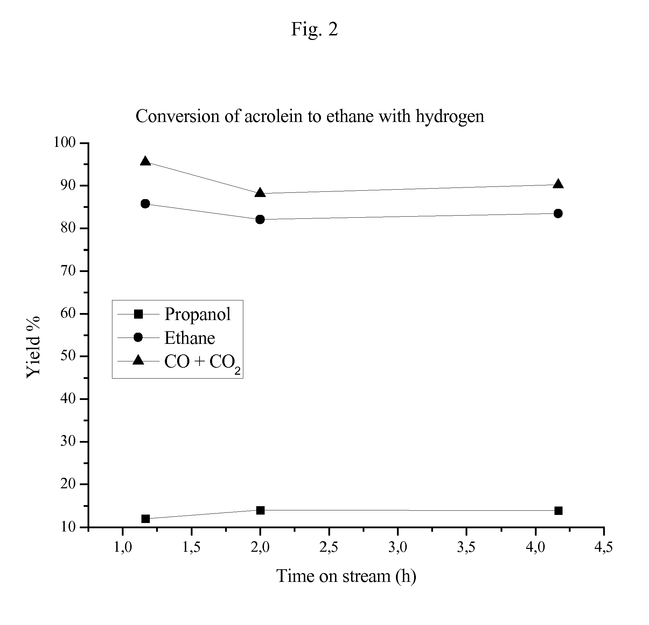

In Fig. 2 the result of a four hour run is shown.

All glycerol was converted to below the limit of detection. The yield of ethane is above 80 %, without any measurable ethene formation. Carbon monoxide plus carbon dioxide should be formed in a 1:1 ratio to ethane, but since there is slightly more CO+CO₂ than ethane formed it is indicated that a small part of the glycerol reacts according to a different path-way. Propanol is the only measurable byproduct and is produced in a yield of 15 %.

### Example 2 -production of ethane without external hydrogen supply

In this example the same conditions and catalysts as in example 1, with the exception that no hydrogen was added, were used.

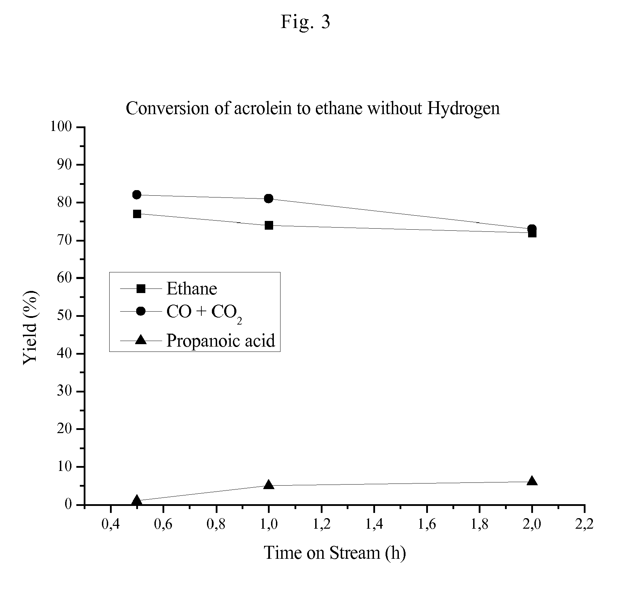

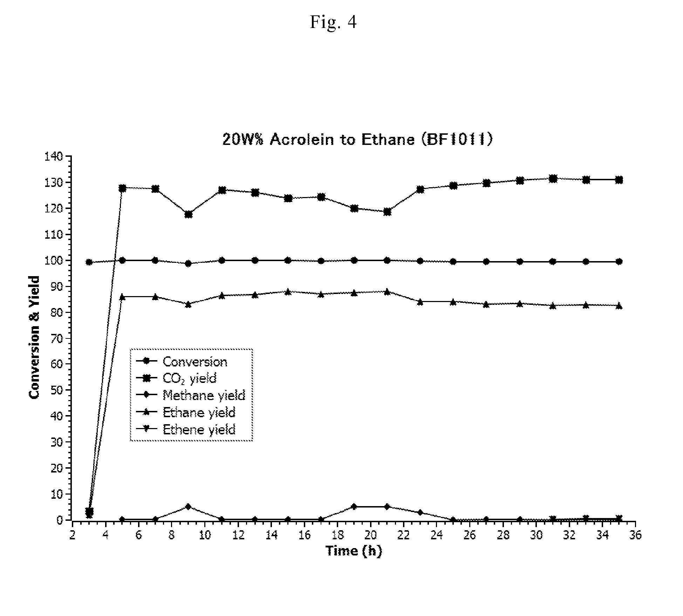

In Fig. 3 and 4 the yield of ethane, CO₂, CO and propanoic acid, in two different runs, are shown.

In spite of the lack of added hydrogen, the main product is ethane, which is produced with high selectivity.

Without being bond to any theory is believed that the hydrogen needed for the hydrogenation is produced internally by reaction of CO with H₂O, forming CO₂ and H₂. The formation of ethane, the high CO₂-content of the gas and the low CO-content of the gas support this hypothesis.

As seen from the second run, cf. Fig. 4, the process is stable over time, only showing minor variation in the yield of the formed species.

### Example 3 - production of ethene

In this example the production of ethene from acrolein was demonstrated. 11 g of a Pd/CeO₂ catalyst was loaded as grains of the size 20-30 mesh in the first reactor 7. The feed was 18 g/h of 10 wt% of acrolein in water. A carrier flow of 100 ml/min of N₂ was added to the stream in the pre-heater 3. The inlet temperature in the first reactor 7 was about 300°C and the total pressure over the reactors 7 to 9 was 5 bars.

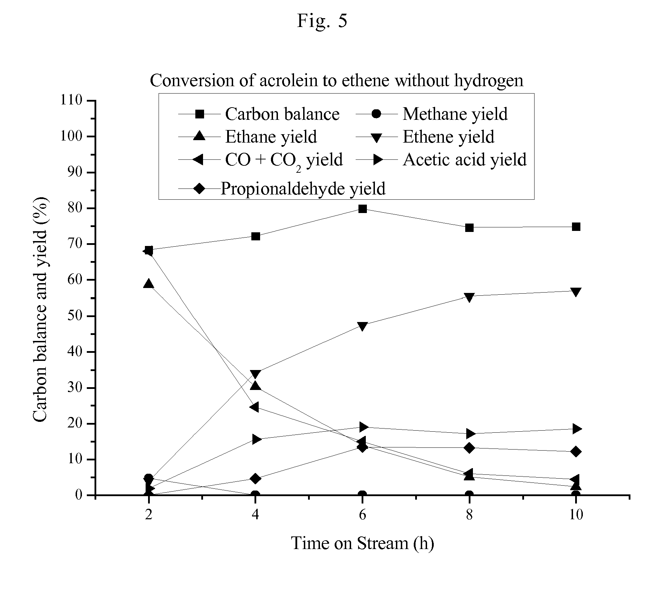

In Fig. 5 the result of a 10 h run is depicted. As shown ethane is formed on a fresh non-reduced catalyst, but the ethane formation decreases and ethene is formed instead.

If it is crucial to have high initial yields of ethane the catalyst may be reduced *in-situ* or *ex-situ* by standard measures.

### Example 4 - production of propene

To the first reactor 7 loaded with 30 g of a dehydration catalyst (WO₃/ZrO₂, grains of the size 20-30 mesh, first catalyst), 20 wt% glycerol in water was fed at a rate of 18 g/h and hydrogen at a flow rate of 400ml/min. The second reactor 8 was loaded with 12 g of a hydrogenation catalyst (Cu/ZnO₂, grains of the size 20-30 mesh, second catalyst) and the third reactor 9 was loaded with 30 g of a dehydration catalyst (WO₃/ZrO₂, grains of the size 20-30 mesh, third catalyst). The total pressure over the system was 5 bars. The inlet temperatures in the reactors 7 to 9 were 280°C, 270°C and 208°C, respectively.

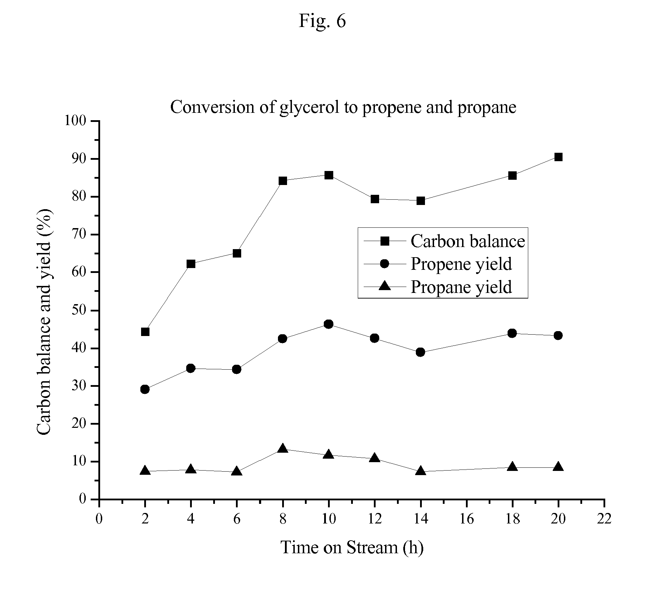

In Fig. 6 the yield of propene, propane and the total carbon balance are shown.

As major by-products, except to propane, are CO₂ and CO, it seems like some de-carbonylation takes place.

### Example 5 - production of propane

To the first reactor 7 loaded with 30 g of a dehydration catalyst (WO₃/ZrO₂, grains of the size 20-30 mesh, first catalyst), 20 wt% glycerol in water was fed at a rate of 18 g/h. A stream of hydrogen (400 ml/min) was also fed to the first reactor 7. The second reactor 8 was loaded with 12 g of a hydrogenation catalyst (Cu/ZnO₂, grains of the size 20-30 mesh, second catalyst). The third reactor 9 was loaded with 30 g of a dehydration catalyst (WO₃/ZrO₂, grains of the size 20-30 mesh, third catalyst) and 12 g of a hydrogenation catalyst (Cu/ZnO₂, grains of the size 20-30 mesh, fourth catalyst). The total pressure over the system was 5 bars. The inlet temperatures in the reactors 7 to 9 were 280°C, 270°C and 208°C, respectively.

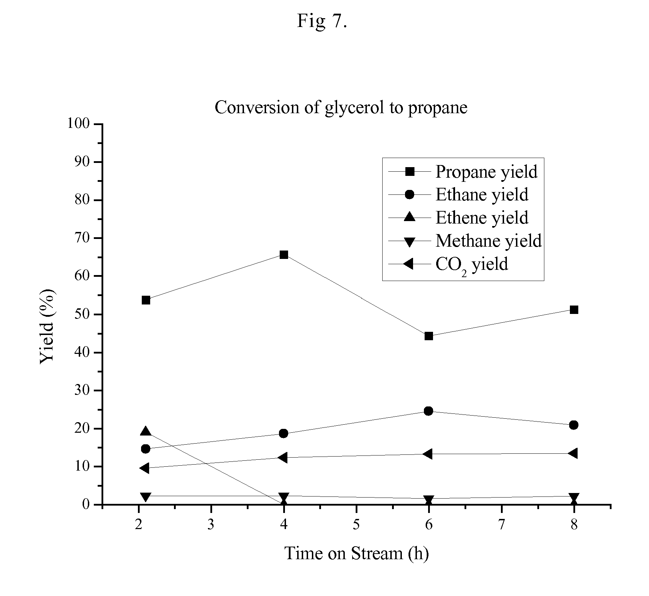

In Fig. 7 the yield of propane, various by-products and the total carbon balance are shown.

As seen from Fig. 7, propane was produced in average in more than 50% yield from glycerol. The major by-product was ethane, which was produced in less than 25% yield. Except to CO₂ all other by-products were only present in very low amounts.

## Claims

1. A method of producing a hydrocarbon comprising two or three carbon atoms from glycerol, wherein the hydrocarbon only comprises carbon and hydrogen atoms, and wherein the method comprises the steps of:
providing glycerol;
converting glycerol to acrolein through use of a first catalyst, catalyzing de-hydration reactions; and
converting the formed acrolein to the hydrocarbon comprising two or three carbon atoms, through use of a second catalyst catalyzing de-carbonylation reactions, or through use of a second catalyst catalyzing hydrogenation reactions and a third catalyst catalyzing de-hydration reactions.

2. The method according to claim 1, wherein the hydrocarbon is ethane or ethene and wherein the second catalyst catalyzes de-carbonylation reactions.

3. The method according to claim 2, wherein the hydrocarbon is ethane and
wherein the second catalyst is selected from catalysts catalyzing water-gas-shift (WGS), hydrogenation and de-carbonylation reactions.

4. The method according to claim 3, wherein the second catalyst comprises Pt on CeO₂.

5. The method according to any of the preceding claims, wherein the first and the second catalysts are present in a first reactor, being the same reactor.

6. The method according to claim 1, further comprising the step of:
providing hydrogen, wherein the hydrocarbon is propane or propene and
wherein the second catalyst catalyzes hydrogenation reactions and the third catalyst catalyzes de-hydration reactions.

7. The method according to claim 6, wherein the hydrocarbon is propane and wherein hydrogen is provided in stoichiometric excess with regard to glycerol.

8. The method according to any of the preceding claims, wherein glycerol is provided as mixture with water and the concentration of glycerol in the mixture of glycerol and water is 1 to 75wt%.

9. The method according to claim 8, wherein the temperature of the glycerol/water-mixture, when provided is 200 to 400°C, and wherein the glycerol is converted to acrolein at a temperature of 200 to 400°C and at a pressure of 2 to 10 bar.

10. The method according to any of the preceding claims, wherein the first catalyst is an acidic catalyst comprising WO₃ supported on ZrO₂.

11. The method according to claim 1, wherein
- said hydrocarbon is ethane;
- said first catalyst is an acidic catalyst;
- said second catalyst is a catalyst comprising Pt on CeO₂;
- glycerol is provided as a mixture with water, such as a mixture having a glycerol concentration of 15 to 30wt%;
- the temperature at which glycerol is converted to acrolein and acrolein to ethane is 250°C to 350°C;
- the pressure at which glycerol is converted to acrolein and acrolein to ethane is 3.5 to 4.5 bar;
- the temperature of the glycerol/water-mixture when provided is 250 to 350 °C; and
- said first and said second catalyst are present in the same reactor.

12. The method according to claim 11, wherein said first catalyst is an acidic catalyst comprising WO₃ on ZrO₂.

13. The method according to claim 1 or 8 to 10, wherein the hydrocarbon is ethene and the second catalyst is catalyst comprising Pd/CeO₂ as the catalyzing specie.

14. The method according to claim 1, wherein:
glycerol is provided as mixture with water in gas phase, said mixture having a temperature of 200 to 400°C;
glycerol is converted to acrolein at a temperature of 200°C to 400°C and at a pressure of 2 to 10 bar;
the method further comprises the step of providing hydrogen in stoichiometric excess with regard to glycerol;
the hydrocarbon is propane; and
acrolein is converted to propane, through use of:
a second catalyst catalyzing hydrogenation reactions, said second catalyst comprising Cu on ZnO₂, to convert acrolein to propanol;
a third acidic catalyst catalyzing de-hydration reactions, to convert propanol to propene; and
a fourth catalyst catalyzing hydrogenation reactions, to convert propene to propane.

15. The method according to claim 14, wherein said third catalyst comprises WO₃ on ZrO₂ and said fourth catalyst is a catalyst comprising a platinum group metal.

## Patentansprüche

1. Verfahren zum Herstellen eines Kohlenwasserstoffs umfassend zwei oder drei Kohlenstoffatome von Glycerin, wobei der Kohlenwasserstoff nur Kohlenstoff- und Wasserstoff-Atome umfasst, und wobei das Verfahren die Schritte umfasst von:
Bereitstellen von Glycerin;
Umwandeln von Glycerin zu Acrolein durch Verwendung eines ersten Katalysators, welcher Dehydratisierungs-Reaktionen katalysiert; und
Umsetzen des gebildeten Acroleins zu dem Kohlenwasserstoff umfassend zwei oder drei Kohlenstoffatome durch Verwendung eines zweiten Katalysators, der Decarbonylierungs-Reaktionen katalysiert, oder durch Verwendung eines zweiten Katalysators, der Hydrierungsreaktionen katalysiert und eines dritten Katalysators, der Dehydratisierungs-Reaktionen katalysiert.

2. Verfahren nach Anspruch 1, wobei der Kohlenwasserstoff Ethan oder Ethen ist und wobei der zweite Katalysator Decarbonylierungs-Reaktionen katalysiert.

3. Verfahren nach Anspruch 2, wobei der Kohlenwasserstoff Ethan ist und wobei der zweite Katalysator ausgewählt ist aus Katalysatoren, welche Wasser-Gas-Verlagerung (water-gas-shift, WGS), Hydrierungs- und Decarbonylierungs-Reaktionen katalysieren.

4. Verfahren nach Anspruch 3, wobei der zweite Katalysator Pt auf CeO₂ umfasst.

5. Verfahren nach einem beliebigen der vorhergehenden Ansprüche, wobei der erste und der zweite Katalysator in einem ersten Reaktor vorliegen, welcher der gleiche Reaktor ist.

6. Verfahren nach Anspruch 1, des Weiteren umfassend den Schritt von:
Bereitstellen von Wasserstoff, wobei der Kohlenwasserstoff Propan oder Propen ist und wobei der zweite Katalysator Hydrierungs-Reaktionen katalysiert und der dritte Katalysator Dehydratisierungs-Reaktionen katalysiert.

7. Verfahren nach Anspruch 6, wobei der Kohlenwasserstoff Propan ist und wobei Wasserstoff in stöchiometrischem Überschuss bezogen auf Glycerin bereitgestellt wird.

8. Verfahren nach einem beliebigen der vorhergehenden Ansprüche, wobei Glycerin als eine Mischung mit Wasser bereitgestellt wird und die Konzentration von Glycerin in der Mischung von Glycerin und Wasser 1 bis 75 Gew.-% beträgt.

9. Verfahren nach Anspruch 8, wobei die Temperatur der Glycerin/Wasser-Mischung, wenn bereitgestellt 200 bis 400 °C ist, und wobei das Glycerin bei einer Temperatur von 200 bis 400 °C und einem Druck von 2 bis 10 bar zu Acrolein umgesetzt wird.

10. Verfahren nach einem beliebigen der vorhergehenden Ansprüche, wobei der erste Katalysator ein saurer Katalysator umfassend auf ZrO₂ geträgertes WO₃ ist.

11. Verfahren nach Anspruch 1, wobei
- der Kohlenwasserstoff Ethan ist;
- der erste Katalysator ein saurer Katalysator ist;
- der zweite Katalysator ein Katalysator umfassend Pt auf CeO₂ ist;
- Glycerin als eine Mischung mit Wasser bereitgestellt wird, wie eine Mischung mit einer Glycerinkonzentration von 15 bis 30 Gew.-%;
- die Temperatur, bei welcher Glycerin zu Acrolein und Acrolein zu Ethan umgesetzt wird, 250 °C bis 350 °C ist;
- der Druck, bei welchem Glycerin zu Acrolein und Acrolein zu Ethan umgesetzt wird, 3,5 bis 4,5 bar ist;
- die Temperatur der Glycerin/Wasser-Mischung wenn bereitgestellt, 250 bis 350 °C ist; und
- der erste und der zweite Katalysator in dem gleichen Reaktor vorliegen.

12. Verfahren nach Anspruch 11, wobei der erste Katalysator ein saurer Katalysator umfassend WO₃ auf ZrO₂ ist.

13. Verfahren nach Anspruch 1 oder 8 bis 10, wobei der Kohlenwasserstoff Ethen ist und der zweite Katalysator ein Katalysator ist umfassend Pd/CeO₂ als die katalysierende Spezies.

14. Verfahren nach Anspruch 1, wobei:
Glycerin als Mischung mit Wasser in Gasphase bereitgestellt wird, wobei die Mischung eine Temperatur von 200 bis 400 °C aufweist;
Glycerin zu Acrolein bei einer Temperatur von 200 °C bis 400 °C und bei einem Druck von 2 bis 10 bar umgesetzt wird;
das Verfahren des Weiteren den Schritt des Bereitstellens von Wasserstoff in stöchiometrischem Überschuss bezogen auf Glycerin umfasst;
der Kohlenwasserstoff Propan ist; und
Acrolein zu Propan umgesetzt wird durch die Verwendung von:
einem zweiten Katalysator, welcher Hydrierungsreaktionen katalysiert, wobei der zweite Katalysator Cu auf ZnO₂ umfasst, um Acrolein zu Propanol umzusetzen;
einem dritten sauren Katalysator, welcher Dehydratisierungs-Reaktionen katalysiert, um Propanol zu Propen umzusetzen; und
einem vierten Katalysator, welcher Hydrierungsreaktionen katalysiert, um Propen zu Propan umzusetzen.

15. Verfahren nach Anspruch 14, wobei der dritte Katalysator WO₃ auf ZrO₂ umfasst und der vierte Katalysator ein Katalysator ist, der ein Platin-Gruppenmetall umfasst.

## Revendications

1. Procédé de production d'un hydrocarbure comprenant deux ou trois atomes de carbone de glycérol, dans lequel l'hydrocarbure comprend uniquement des atomes de carbone et d'hydrogène, et le procédé comprenant les étapes de :
fourniture de glycérol ;
conversion de glycérol en acroléine par utilisation d'un premier catalyseur, catalysant des réactions de déshydratation ; et
conversion de l'acroléine formée en hydrocarbure comprenant deux ou trois atomes de carbone, par utilisation d'un deuxième catalyseur catalysant des réactions de décarbonylation, ou par utilisation d'un deuxième catalyseur catalysant des réactions d'hydrogénation et un troisième catalyseur catalysant des réactions de déshydratation.

2. Procédé selon la revendication 1, dans lequel l'hydrocarbure est l'éthane ou l'éthène et dans lequel le deuxième catalyseur catalyse des réactions de décarbonylation.

3. Procédé selon la revendication 2, dans lequel l'hydrocarbure est l'éthane et dans lequel le deuxième catalyseur est choisi parmi des catalyseurs catalysant des réactions de déplacement eau-gaz (WGS), d'hydrogénation et de décarbonylation.

4. Procédé selon la revendication 3, dans lequel le deuxième catalyseur comprend Pt on sur CeO₂.

5. Procédé selon l'une quelconque des revendications précédentes, dans lequel les premier et deuxième catalyseurs sont présents dans un premier réacteur, étant le même réacteur.

6. Procédé selon la revendication 1, comprenant en outre l'étape de :
fourniture d'hydrogène, dans lequel l'hydrocarbure étant le propane ou le propène et dans lequel le deuxième catalyseur catalyse des réactions d'hydrogénation et le troisième catalyseur catalyse des réactions de déshydratation.

7. Procédé selon la revendication 6, dans lequel l'hydrocarbure est le propane et dans lequel l'hydrogène est fourni en excès stoechiométrique par rapport au glycérol.

8. Procédé selon l'une quelconque des revendications précédentes, dans lequel le glycérol est fourni sous la forme d'un mélange avec de l'eau et la concentration de glycérol dans le mélange de glycérol et d'eau est de 1 à 75 % en poids.

9. Procédé selon la revendication 8, dans lequel la température du mélange glycérol/eau, lorsqu'il est fourni est de 200 à 400 °C, et dans lequel le glycérol est converti en acroléine à une température de 200 à 400 °C et à une pression de 2 à 10 bar.

10. Procédé selon l'une quelconque des revendications précédentes, dans lequel le premier catalyseur est un catalyseur acide comprenant WO₃ sur support de ZrO₂.

11. Procédé selon la revendication 1, dans lequel
- ledit hydrocarbure est l'éthane ;
- ledit premier catalyseur est un catalyseur acide ;
- ledit deuxième catalyseur est un catalyseur comprenant Pt sur CeO₂ ;
- le glycérol est fourni sous la forme d'un mélange avec de l'eau, tel qu'un mélange ayant une concentration de glycérol de 15 à 30 % en poids ;
- la température à laquelle le glycérol est converti en acroléine et l'acroléine en éthane est de 250 °C à 350 °C ;
- la pression à laquelle le glycérol est converti en acroléine et l'acroléine en éthane est de 3,5 à 4,5 bar ;
- la température du mélange glycérol/eau lorsqu'il est fourni est de 250 à 350 °C ; et
- ledit premier et ledit deuxième catalyseur sont présents dans le même réacteur.

12. Procédé selon la revendication 11, dans lequel ledit premier catalyseur est un catalyseur acide comprenant WO₃ sur ZrO₂.

13. Procédé selon la revendication 1 ou 8 à 10, dans lequel l'hydrocarbure est l'éthène et le deuxième catalyseur est le catalyseur comprenant Pd/CeO₂ en tant qu'espèce de catalyseur.

14. Procédé selon la revendication 1, dans lequel :
le glycérol est fourni sous la forme d'un mélange avec de l'eau en phase gazeuse, ledit mélange ayant une température de 200 à 400 °C ;
le glycérol est converti en acroléine à une température de 200 °C à 400 °C et à une pression de 2 à 10 bar ;
le procédé comprend en outre l'étape de fourniture d'hydrogène en excès stoechiométrique par rapport au glycérol ;
l' hydrocarbure est le propane ; et
l'acroléine est convertie en propane, par utilisation de :
un deuxième catalyseur catalysant des réactions d'hydrogénation, ledit deuxième catalyseur comprenant Cu sur ZnO₂, pour convertir l'acroléine en propanol ;
un troisième catalyseur acide catalysant des réactions de déshydratation, pour convertir le propanol en propène ; et
un quatrième catalyseur catalysant des réactions d'hydrogénation, pour convertir le propène en propane.

15. Procédé selon la revendication 14, dans lequel ledit troisième catalyseur comprend WO₃ sur ZrO₂ et ledit quatrième catalyseur est un catalyseur comprenant un métal du groupe du platine.
